(19) Europäisches Patentamt
European Patent Office
Office européen des brevets

(11) **EP 1 870 063 A2**

(12) **EUROPÄISCHE PATENTANMELDUNG**

(43) Veröffentlichungstag:
26.12.2007 Patentblatt 2007/52

(51) Int Cl.:
*A61F 9/02* *(2006.01)*

(21) Anmeldenummer: 07405170.7

(22) Anmeldetag: **15.06.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK YU**

(30) Priorität: **20.06.2006 CH 9942006**

(71) Anmelder: **SPERIAN Welding Protection AG 9630 Wattwil (CH)**

(72) Erfinder:
• **Gerfin, Tobias**
**8942 Oberrieden (CH)**
• **Cottier Kaspar**
**8645 Jona (CH)**

(74) Vertreter: **Frei Patent Attorneys**
**Frei Patentanwaltsbüro**
**Postfach 1771**
**8032 Zürich (CH)**

(54) **Blendschutzeinheit für eine tragbare Blendschutzvorrichtung**

(57)     Eine Blendschutzeinheit (1) für eine tragbare Blendschutzvorrichtung weist ein optisches Blendschutzfilter (3) und mindestens einen Sensor (5) zur Erfassung von einfallendem Licht und zur Ansteuerung der Durchlässigkeit des Filters (3) auf. Dabei weist die Blendschutzeinheit (1) ein Lichterfassungselement (4), welches einfallendes Licht auf einem Erfassungsbereich (12) erfasst und dem mindestens einen Sensor (5) zuführt, auf. Das Lichterfassungselement (4) erfasst vorzugsweise Licht von mehreren, voneinander beabstandeten Bereichen an einer Vorderseite der Blendschutzeinheit (1). Mittel (8) zum Einkoppeln von Licht in das Lichterfassungselement (4) sind voneinander beabstandet.

Fig. 4

EP 1 870 063 A2

**Beschreibung**

Die Erfindung bezieht sich auf das Gebiet der Blendschutzvorrichtungen, wie sie beispielsweise bei Schweisserschutzmasken Verwendung finden, und insbesondere auf eine Blendschutzeinheit für eine tragbare Blendschutzvorrichtung gemäss dem Oberbegriff des Patentanspruches 1.

**STAND DER TECHNIK**

Blendschutzeinheiten für tragbare Blendschutzvorrichtungen wie Schutzmasken und Schutzbrillen sind allgemein bekannt. Moderne Blendschutzvorrichtungen weisen elektrooptische Filter auf, beispielsweise mit einem Flüssigkristallelement, deren Durchlässigkeit automatisch oder manuell angepasst wird. Dazu kann mit einem Sensor des einfallenden Lichts gemessen werden. Es besteht ein gewisses Risiko, dass die Oberfläche des Sensors, oder ein Schutzfenster vor dem Sensor, verschmutzt wird oder vorübergehend im Schatten des zu detektierenden Lichtes liegt. In solchen Fällen kann der Sensor es versäumen, einen Schweissvorgang zu detektieren, was wiederum zu Schädigungen am Auge des Benutzers führen kann. Einzelne Hersteller sind deshalb dazu übergegangen, ihre Blendschutzeinheiten mit mehreren, beispielsweise vier Sensoren auszurüsten.

US 2005/0007667 A1 offenbart eine Blendschutzvorrichtung, in welcher Lichtleiter einfallendes Licht zu einem Sensor führen. Eine sägezahnartige Struktur des Lichtleiters soll Licht in diesen einkoppeln, führt aber auch zum Auskoppeln des Lichtes.

US 2005/0097648 A1 zeigt eine Blendschutzvorrichtung, bei welcher ein UV-Wandler durch einen Lichtwellenleiter von einem Sensor abgesetzt ist: UV-Licht wird in einer fluoreszierenden Konverterschicht in sichtbares Licht im Nah-InfrarotBereich gewandelt, über eine Optik auf einen Lichtwellenleiter und so zu einem Sensor geführt.

**DARSTELLUNG DER ERFINDUNG**

Es ist deshalb Aufgabe der Erfindung, eine Blendschutzeinheit für eine tragbare Blendschutzvorrichtung der eingangs genannten Art zu schaffen, welche eine verbesserte und robustere Lichterfassung bei gleichzeitig geringem technischen Aufwand erlaubt. Eine weitere Aufgabe der Erfindung ist es, eine einfach anpassbare Lichterfassung bereitzustellen, welche nach Massgabe verschiedener Arbeitssituationen eingestellt werden kann.

Diese Aufgabe löst eine Blendschutzeinheit für eine tragbare Blendschutzvorrichtung mit den Merkmalen des Patentanspruches 1.

Die Blendschutzeinheit für eine tragbare Blendschutzvorrichtung weist einen optischen Blendschutzfilter und mindestens einem Sensor zur Erfassung von einfallendem Licht und zur Ansteuerung der Durchlässigkeit des Filters nach Massgabe der erfassten Lichtmenge auf. Dabei weist die Blendschutzeinheit ein Lichterfassungselement auf, welches einfallendes Licht auf einem Erfassungsbereich erfasst und dem mindestens einen Sensor zuführt. Die Fläche des Erfassungsbereichs ist dabei grösser als die Fläche des Sensors. Insbesondere sind Mittel zum Einkoppeln von Licht in das Lichterfassungselement voneinander beabstandet. Dadurch wird es möglich, dass diese Mittel zum Einkoppeln von Licht sich nicht gegenseitig behindern.

Die Blendschutzeinheit wird typischerweise als Blendschutzkassette in einer Schutzmaske wie eine Schweisserschutzmaske oder in einer Schutzbrille verwendet. Blendschutzkassetten sind kompakte Baueinheiten mit Filter, Energieversorgung, Sensor(en) und Steuerelektronik in einem gemeinsamen Gehäuse, und in der Regel werkzeugfrei in einer Maske montierbar respektive herausnehmbar.

Das Lichterfassungselement erfasst vorzugsweise Licht von mehreren, voneinander beabstandeten Bereichen an einer Vorderseite der Blendschutzeinheit. Dabei umschliesst die konvexe Hülle des Erfassungsbereiches eine Fläche, welche mindestens das zwanzigfache oder mindestens das zehnfache der Fläche des mindestens einen Sensors beträgt. Durch diese grössere Abdeckung wird die Messung der einfallenden Lichtmenge weniger von lokalen Verdunkelungen oder Verschmutzungen abhängig. Es kann im Weiteren der Erfassungsbereich auch mehrere voneinander getrennte Bereiche umfassen, von denen das einfallende Licht zum selben Sensor geleitet wird, wobei also andere Bereiche des Lichterfassungselementes bezüglich des einfallenden Lichtes abgedeckt sind. Trotzdem sind dabei also mehrerer Erfassungsbereiche voneinander beabstandet, so dass, wenn der eine verdunkelt ist, der andere noch immer ausreichend Licht erfasst, um das Filter mit Hilfe von beispielsweise einer Flackerschaltung zuverlässig ansteuern zu können.

Die Fläche des Erfassungsbereiches beträgt vorzugsweise mehr als das fünffache oder mindestens das doppelte der Fläche eines der zugeordneten Sensoren, und insbesondere mehr als das zehnfache oder mehr als das zwanzigfache oder fünfzigfache.

Das Lichterfassungselement weist vorzugsweise Mittel zum Umlenken des einfallenden Lichtes zum mindestens einen Sensor durch interne Reflexion auf. Diese Mittel sind beispielsweise lichtleitende Strukturen, insbesondere Einbuchtungen am Lichterfassungselement. Diese dienen insbesondere zum Einkoppeln oder Einlenken von Licht von aussen in das Lichterfassungselement, und zum Auskoppeln oder Auslenken von Licht zu einem oder mehreren Sensoren. In einer bevorzugten Ausführungsform der Erfindung sind die Mittel zum Umlenken Austrittsprismen und/oder Eintrittsprismen, welche sich von einem äusseren Teil, insbesondere von einer Aussenseite, des Lichterfassungselementes her in das Lichterfassungselement hinein erstrecken. Damit ist eine konstruktiv besonders einfache Gestaltung der Lichtumlenkung möglich, indem das Licht durch interne Totalreflexion durch das Lichterfassungselement zu den Sensoren geleitet wird. In einer weiteren Ausführungsform der Erfindung ist das Lichterfassungselement an den Aussenseiten gegen innen verspiegelt, um die Lichtführung durch totale interne Reflexion zu verbessern. Die Erfassungsbereiche und die Übergangsbereiche zu den Sensoren sind dabei aber nicht verspiegelt.

Vorzugsweise ist das Lichterfassungselement einstückig geformt, und ist am Lichterfassungselement für zwei oder mehr Sensoren jeweils ein eigenes Mittel zum Auskoppeln des einfallenden Lichtes zum jeweiligen Sensor ausgebildet. Auch damit ist eine einfache Konstruktion realisierbar, indem mit einem einzigen Element mehrere Sensoren versorgt werden können.

In einer weiteren bevorzugten Ausführungsform der Erfindung weisen die Strukturen zum Einkoppeln von Licht in das Lichterfassungselement einen Abstand von mindestens dem doppelten der lokalen Dicke des Lichterfassungselementes zueinander auf. Die Dikke wird in die Richtung entlang einer Referenzeintrittsrichtung gemessen, siehe unten. Der Begriff "lokale Dikke" meint die Dicke im Bereich der Struktur zum Einkoppeln. Damit ist es möglich, die Umlenkung des erfassten Lichtes zu bewirken, ohne dass das Licht gleich durch eine anschliessende weitere Struktur zum Einkoppeln abgeschwächt oder in die falsche Richtung ausgekoppelt wird.

Ferner wird vorzugsweise durch mindestens eine Einbuchtung oder einen Hohlraum Licht aus zwei entgegengesetzten Richtungen aus dem Lichterfassungselement zum selben Sensor ausgekoppelt wird, wobei das Lichterfassungselement im Bereich dieser Einbuchtung respektive dieses Hohlraums einstückig geformt ist. Auch dadurch wird die Konstruktion vereinfacht.

Vorzugsweise weist mindestens eines der Eintrittsprismen mindestens eine Ablenkfläche auf, die gegenüber einer Referenzeintrittsrichtung um einen Neigungswinkel von 0 bis 90° und insbesondere von 10° bis 70° und vorzugsweise von 35° bis 50° geneigt ist. Dabei verläuft die Referenzeintrittsrichtung parallel zu einer Flächennormalen einer lichterfassenden Oberfläche des Lichterfassungselementes.

Die Eintrittsprismen lenken das Licht in eine Umlenkrichtung. Die Umlenkrichtung ist die Richtung, die vom entsprechenden Eintrittsprisma zu einem zugeordneten Sensor oder Austrittsprisma führt. Im Falle eines langgestreckten Lichterfassungselementes ist dies die Längsrichtung entlang des Lichterfassungselementes. In einer weiteren bevorzugten Ausführungsform der Erfindung ist die Prismenfläche auch um eine Achse parallel zur Blickrichtung verdreht. Damit kann die Richtungsempfindlichkeit der Einrichtung kontrolliert werden.

Vorzugsweise ist dabei mindestens ein Austrittsprisma einem der Sensoren gegenüber angeordnet, und sind eines oder mehrere der Eintrittsprismen entlang des Lichterfassungselementes auf derselben Seite des Lichterfassungselementes wie der mindestens eine Sensor angeordnet, und von dem mindestens einen Sensor beabstandet angeordnet. Es ist aber auch möglich, mindestens einen der Sensoren an einer der Stirnseiten des Lichterfassungselementes anzuordnen (und optional mit diesem zu verkleben), entweder mit der Blickrichtung des Sensors parallel zur Längsausdehnung des Lichterfassungselementes oder aber senkrecht dazu, und mit einer Umlenkung des Lichtes durch Abschrägen des Endes des Lichterfassungselementes. Die Austrittsprismen können abgedeckt sein, respektive im Lichterfassungselement eingeschlossen sein, und somit einen Hohlraum bilden. Durch die Anordnung eines Austrittsprismas als Teil des Lichterfassungselementes kann das Lichterfassungselement Licht von zwei Seiten her in den Sensor leiten und gleichwohl einstückig ausgebildet sein.

Das Lichterfassungselement ist vorzugsweise ein linear ausgedehntes oder stabartiges Prisma aus einem lichtdurchlässigen Material. Das Lichterfassungselement ist also länglich und beispielsweise ca. 5 bis 15 bis 20 mal so lang wie breit.

In einer Blendschutzkassette erstreckt sich das Lichterfassungselement vorzugsweise linear entlang einer Seite des Filters, und insbesondere entlang mindestens zwei Dritteln bis der ganzen Länge dieser Seite.

In einer bevorzugten Ausführungsform der Erfindung ist das Lichterfassungselement entlang einer im vorgesehenen Betrieb im wesentlichen senkrechten Achse bezüglich der Sensoren asymmetrisch und insbesondere nach unten verschoben angeordnet. Die horizontale, längs des Lichterfassungselementes verlaufende Symmetrieachse ist also nicht auf gleicher Höhe wie die Sensoren angeordnet, sondern etwas tiefer. Entsprechend ist auch ein Gehäuserand, der den Erfassungsbereich des Lichterfassungselementes umschliesst und als Abschirmung oder Abdeckung wirkt, nach unten verschoben angeordnet. Dadurch ist die gesamte Anordnung vor allem auf Licht, das von unten her einfällt, empfindlich.

Der mindestens eine Sensor ist vorzugsweise durch einen Klebstoff am Lichterfassungselement angeklebt. Dabei weist der Klebstoff vorzugsweise mindestens annähernd denselben Brechungsindex wie das Material des Lichterfassungselementes auf. Dadurch liegt kein Luftspalt zwischen dem Lichterfassungselement und dem Sensor vor, was den Lichtübergang verbessert, insbesondere weil keine Totalreflexion am Übergang zum Sensor stattfindet. In einer weiteren bevorzugten Ausführungsform der Erfindung ist das Lichterfassungselement im Bereich des Lichtaustrittes zum Sensor aufgerauht, um die Auskopplung des Lichtes zu verbessern.

In einer bevorzugten Ausführungsform der Erfindung ist das Lichterfassungselement im Erfassungsbereich als Linse ausgebildet. Die Linse kann eine refraktiv und/oder diffraktiv wirkende Linse sein. Eine refraktive Linse ist vorzugsweise zylindrisch oder rotationssymmetrisch geformt.

Das Lichterfassungselement, zusammen mit allfälligen Linsenfunktionen, ist vorzugsweise einstückig aus einem transparenten Kunststoff wie Acrylglas, Polycarbonat etc. geformt. Das Lichterfassungselement kann auch durch einen durchsichtigen Klebstoff gebildet sein, der in einen Hohlraum oder eine Aufnahmeöffnung der Blendschutzeinheit eingebracht und ausgehärtet wird. Dieser Hohlraum ist somit als Negativ oder Giessform für das Lichterfassungselement geformt.

In einer weiteren bevorzugten Ausführungsform der Erfindung weist die Blendschutzeinheit mindestens zwei Linsen auf, welche verschiebbar angeordnet sind und in einer ersten und einer zweiten Position im Strahlengang des einfallenden Lichtes anordenbar sind. Dabei resultiert in der ersten Position ein erster Öffnungswinkel des Sensors und in der zweiten Position ein zweiter, unterschiedlicher Öffnungswinkel des Sensors. Dieser Öffnungswinkel oder Ansprechwinkel oder Blickwinkel ist der Raumwinkel, unter welchem einfallendes Licht dem Sensor zugeführt wird.

Die verschiebbaren Linsen können entweder nur einem einzelnen Sensor zugeordnet sein, oder es können mehrere Linsen jeweils vor mehreren Erfassungsbereichen eines Lichterfassungselementes angeordnet sein. Solche Sätze von mehreren Linsen können wiederum einzeln oder alle gemeinsam verschiebbar sein. Im letzteren Fall sind die mehreren Linsen vorzugsweise an einem gemeinsamen Linsenträger angeordnet. Durch Bewegung des Linsenträgers wird gleichzeitig für mehrere Sensoren (oder Erfassungsbereiche) jeweils eine von mehreren Linsen eines Linsensatzes, der dem Sensor (oder dem Erfassungsbereich) zugeordnet ist, vor den Sensor (oder den Erfassungsbereich) geschoben.

In einer bevorzugten Ausführungsform der Erfindung weist eine Blendschutzeinheit ein optischen Blendschutzfilter und mindestens zwei Sensoren zur Erfassung von einfallendem Licht und zur Ansteuerung der Durchlässigkeit des Filters nach Massgabe der erfassten Lichtmenge auf. Dabei ist jedem der Sensoren ein lichtführendes Element zugeordnet, und verleihen die lichtführenden Elemente jeweils den zugeordneten Sensoren unterschiedliche Öffnungswinkel.

Vorzugsweise ist mindestens eines der lichtführenden Elemente eine Blende mit einem Blendenrand, wobei der Blendenrand entsprechend einem vorgegebenen Öffnungswinkel geformt ist. Alternativ oder in Kombination damit ist mindestens eines der lichtführenden Elemente eine Linse. Zudem liegt vorzugsweise eine Verarbeitungselektronik vor, welche wahlweise das Signal eines der mindestens zwei Sensoren zur Ansteuerung des Filters heranzieht. Damit kann ohne mechanische Massnahmen der Blickwinkel an die Arbeitssituation angepasst werden.

Eine Blendschutzeinheit oder eine Blendschutzkassette gemäss der Erfindung weist vorzugsweise, auch wenn sie nicht mit einem Lichterfassungselement ausgerüstet ist, eine Aussparung auf, in welche das Lichterfassungselement eingesetzt werden kann. So kann die Blendschutzeinheit ohne konstruktive Veränderung sowohl mit als auch ohne das Lichterfassungselement betrieben werden. Auch ist es möglich, unterschiedliche Lichterfassungselemente mit denselben Aussenmassen vorzusehen, von denen jeweils nach Massgabe der Anzahl von Sensoren und anderen Parametern wie Sichtbereich etc. eines in die Aussparung der Blendschutzeinheit eingesetzt wird.

In einer anderen bevorzugten Ausführungsform der Erfindung umringt das Lichterfassungselement ein grossen Teil oder den gesamten Umfang des Filters. Dabei kann Licht, das in einem Bereich an einer ersten Seite des Filters in das Lichterfassungselement eintritt um die Ecke zu einem Sensor an einer zweiten Seite des Filters geleitet werden. Dazu sind die Ecken des Lichterfassungselementes vorzugsweise in einem Winkel von 45° angeschrägt (bei einer Ansicht senkrecht auf das Filter respektive auf eine Frontfläche der Blendschutzeinheit).

In einer weiteren bevorzugten Ausführungsform der Erfindung ist das Lichterfassungselement ausserhalb der Blendschutzkassette angebracht, also nicht im selben Gehäuse wie das Filter und die Sensoren. Im Betriebszustand weist aber das Lichterfassungselement eine definierte Position bezüglich der Sensoren der Blendschutzkassette auf. Das Lichterfassungselement kann in dieser Ausführungsform entweder als separates Teil am zugehörigen Helm, Handschild oder sonstigem Halter angebracht sein, oder in ein transparentes Element wie einer Vorsatzscheibe, wie sie zum Schutz des Filters vor Verschmutzung verwendet wird, integriert sein, und insbesondere einstückig mit der Scheibe geformt sein.

In weiteren bevorzugten Ausführungsformen der Erfindung weist das Lichterfassungselement Konversionsmittel zum Umwandeln von Ultraviolett-Strahlung in sichtbares Licht oder in Infrarot-Strahlung auf. Dies kann durch eine Beschichtung der Eintritts- oder Austrittsflächen geschehen, oder indem das Material des Lichterfassungselements durchgehend mit einem Farbstoff, welche diese Konversion realisiert, versetzt wird.

Eine solche Blendschutzeinheit weist also eine Basis-Blendschutzeinheit mit dem Filter und dem mindestens einen Sensor, vorzugsweise in einem gemeinsamen Gehäuse, auf, wobei das Lichterfassungselement ausserhalb der Basis-Blendschutzeinheit angeordnet ist. Das Lichterfassungselement kann so als separates Teil einzeln ersetzt werden. Wenn es in einer Vorsatz- oder Schutzscheibe integriert ist, kann es zusammen mit dieser ersetzt werden. Das Lichterfassungselement kann sinngemäss gemäss analogen Ausführungsvarianten von Lichterfassungselementen, wie sie bisher beschrieben wurden, ausgestaltet sein.

Weitere bevorzugte Ausführungsformen gehen aus den abhängigen Patentansprüchen hervor.

## KURZE BESCHREIBUNG DER ZEICHNUNGEN

Im folgenden wird der Erfindungsgegenstand anhand von bevorzugten Ausführungsbeispielen, welche in den beiliegenden Zeichnungen dargestellt sind, näher erläutert. Es zeigen jeweils schematisch:

Figur 1　　eine Schweisserschutzmaske;

Figur 2　　eine Blendschutzeinheit;

Figur 3　　eine Ansicht eines Lichterfassungselementes;

Figur 4　　einen Querschnitt eines Lichterfassungselementes in einer Blendschutzeinheit;

Figur 5　　einen Strahlengang in einem Lichterfassungselement;

Figur 6　　den Strahlengang an einem Eintrittsprisma;

Figur 7　　verschiedene Formen von Eintrittsprismen;

Figur 8　　verschiedene Formen von Austrittsprismen;

Figur 9　　verschiedene Ecken an Lichterfassungselementen;

Figur 10　　eine seitliche Ansicht eines Teils einer Blendschutzeinheit

Figur 11　　eine Ausführungsform der Erfindung mit angeformten Linsen und teilweise abgedecktem Lichterfassungselement;

Figur 12　　eine Lichterfassung mit verschiebbaren Linsen;

Figur 13　　eine Ausführungsform mit reflektierenden Oberflächen;

Figur 14　　eine Lichtführung durch vorgelagerte Blenden;

Figur 15　　eine Lichtführung durch jeweils den Sensoren zugeordnete Linsen; und

Figur 16　　eine bevorzugte Ausführungsform der Erfindung mit einem Lichterfassungselement, welches getrennt und ausserhalb von einer Blendschutzkassette angeordnet ist.

Die in den Zeichnungen verwendeten Bezugszeichen und deren Bedeutung sind in der Bezugszeichenliste zusammengefasst aufgelistet. Grundsätzlich sind in den Figuren gleiche Teile mit gleichen Bezugszeichen versehen.

## WEGE ZUR AUSFÜHRUNG DER ERFINDUNG

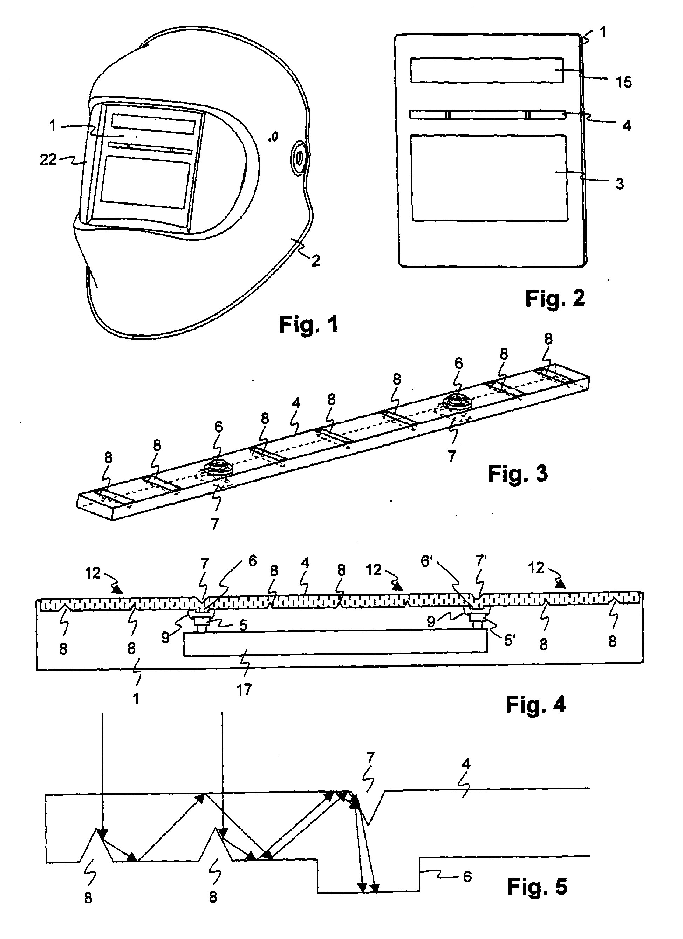

**Figur 1** zeigt eine Schweisserschutzmaske mit einer eingesetzten Blendschutzeinheit 1, hinter einer vorzugsweise auswechselbaren Schutzscheibe 22. **Figur 2** zeigt eine einzelne Blendschutzeinheit 1 mit einem Filter 3 und einem Lichtleiter oder Lichterfassungselement 4. Das Filter 3 ist beispielsweise ein LCD-Filter und wird durch eine in der Blendschutzeinheit 1 integrierte Steuerelektronik angesteuert, d.h. nach Massgabe von empfangenem Licht von einem Lichtbogen automatisch abgedunkelt. Zur Stromversorgung der Steuerelektronik kann die Blendschutzeinheit 1 mit Solarzellen 15 und/ oder einer Batterie ausgerüstet sein.

**Figur 3** zeigt eine perspektivische Ansicht eines Lichterfassungselementes 4 mit Austrittsprismen 7 und Eintrittsprismen 8 zur Führung des einfallenden Lichtes. Die Austrittsprismen 7 liegen an den den Sensoren 5, 5' gegenüberliegenden Flächen des Lichterfassungselementes 4, und die Eintrittsprismen 8 liegen an den den Sensoren zugewandten Flächen. Das Lichterfassungselement 4 besteht aus einem lichtdurchlässigen Material wie einem Acrylglas (mit einem Brechungsindex von rund 1.5) oder einem vorzugsweise UV-resistenten Polycarbonat. Es liegen zwei Austrittsprismen 7 vor, es sind dem Lichterfassungselement 4 also zwei Sensoren 5, 5' zugeordnet. in anderen Ausführungsformen der Erfindung können dies auch noch mehr als zwei Sensoren 5, 5' sein. Vorzugsweise ist das Material getönt, so dass die dahinter angeordnete Elektronik nicht sichtbar ist.

**Figur 4** zeigt schematisch einen Querschnitt eines Lichterfassungselementes 4 in einer Blendschutzeinheit 1. Es sind wiederum die Austrittsprismen 7 und Eintrittsprismen 8 zu sehen, sowie die Sensoren 5, 5', welche den Austrittsprismen 7 gegenüberliegend angeordnet sind. Das Lichterfassungselement 4 weist im Bereich der Sensoren 5, 5' Ausformungen 6, 6' auf, die sich in Richtung der Sensoren 5, 5' erstrecken und das Licht zu den Sensoren 5, 5' leiten. Die Sensoren 5, 5' sind durch Klebstoff 9 mit den Ausformungen 6, 6' verbunden. Die Sensoren 5, 5' sind elektrisch mit der Auswerte- und Steuerelektronik 17 verbunden, die aber im folgenden nicht mehr dargestellt wird.

Beispielhafte Masse für das Lichterfassungselement 4 sind: Länge: 100mm; Breite: 8mm; Höhe 2mm; Neigungswinkel der Eintrittsprismen: 50° für aussenliegende Eintrittsprismen 8; 35° für bei den Sensoren 5, 5' liegende Eintrittsprismen 8; Winkel der Austrittsprismen: 90°; Abstand der Eintrittsprismen 14mm; Abstand zwischen den Sensoren 50mm. Dadurch ist die Fläche des Erfassungsbereiches rund 80 bis 100 mal so gross wie die Fläche eines Sensors, also 40 bis 50 mal so gross wie die Summe der Fläche der beiden parallel verwendeten Sensoren. Die Eintrittsprismen sind also voneinander beabstandet, d.h. dass zwischen Bereichen des Lichterfassungselements 4 mit Eintrittsprismen 8 auch Bereiche ohne Eintrittsprismen 8 angeordnet sind.

**Figur 5** zeigt vergrössert und schematisch einen Strahlengang in einem Teil eines Lichterfassungselementes 4, angedeutet durch Pfeile. Von oben, d.h. von der Vorderseite der Blendschutzeinheit 1 her einfallendes Licht durchquert den Körper des Lichterfassungselementes 4. Ein Teil des Lichtes wird durch totale interne Reflexion an den Innenseiten der Eintrittsprismen 8 seitlich abgelenkt und darauf weiter durch totale interne Reflexion innerhalb des Lichterfassungselementes 4 in der Längsrichtung des Lichterfassungselementes 4 weitergeführt. Im Bereich eines der Sensoren 5, 5' wird das Licht durch die Austrittsprismen 7 nach unten, zu den Sensoren 5, 5', abgelenkt. Es wird klar, dass aufgrund der Beabstandung der Eintrittsprismen 8 der Strahlenverlauf innerhalb des Lichtleiters verbessert wird - würden die Eintrittsprismen unmittelbar und ohne Abstand aufeinander folgen, so würde das Licht zum Teil wie bei einem Retroreflektor so abgelenkt, dass es im steilen Winkel auf die Vorderseite treffen und so den Lichtleiter wieder verlassen würde.

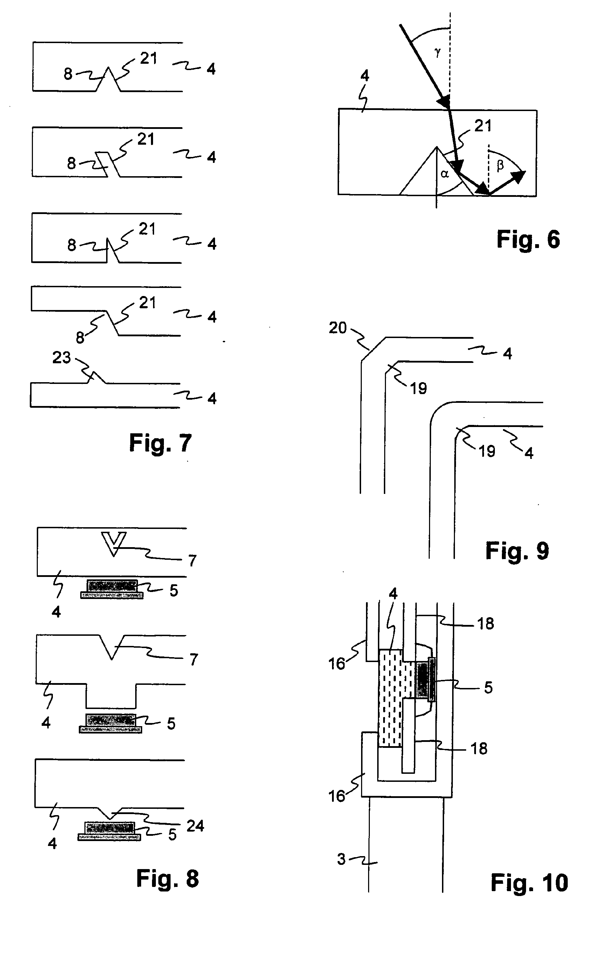

**Figur 6** zeigt den Strahlengang an einem Eintrittsprisma, und wie das Licht durch zweifache Totalreflexion eingekoppelt wird. Der Neigungswinkel $\alpha$ ist der Winkel zwischen der reflektierenden Prismenebene und einer Flächennormalen der Ebene, durch welche das Licht in das Erfassungselement 4 eintritt. Der Eintrittswinkel des Lichtes und der Winkel innerhalb des Erfassungselementes 4, beide ebenfalls bezüglich dieser Flächennormalen, sind mit $\gamma$ respektive $\beta$ bezeichnet. Es ergibt sich, dass

$$\beta = 2\alpha + \text{asin} \ (\sin\gamma \ / \ n)$$

wobei n der Brechungsindex im Erfassungselement 4 ist. Unter Berücksichtigung, dass die innere Totalreflexion im Bereich $90° > \beta > \text{asin}(1/n)$ stattfindet, wird durch Anpassen von $\alpha$ der Ansprechbereich des Prismas 8 eingestellt. Der Ansprechbereich ist gleich jenem Winkelbereich, unter welchem das Licht einfallen muss, um durch das Prima 8 in den Lichtleiter 4 eingekoppelt zu werden. Um einen grösseren Ansprechbereich zu erhalten, können auch mehrere Prismen mit unterschiedlichen Neigungswinkeln eingesetzt werden, beispielsweise mehrere erste Eintrittsprismen 8 mit einem ersten Neigungswinkel und mehrere zweite Eintrittsprismen 8 mit einem zweiten Neigungswinkel.

**Figur 7** zeigt verschiedene Formen von Eintrittsprismen, und zwar, von oben nach unten: Ein Prisma mit gegenüberliegenden, zueinander hin geneigten Flächen; ein Prisma mit zwei parallelen Flächen; ein Prisma mit einer geneigten Fläche und einer (bezüglich der lichterfassenden Eintrittsfläche) senkrechten Fläche; ein Prisma mit einer geneigten Fläche und einer zur Eintrittsfläche parallelen Fläche; sowie ein vorderseitiges Prisma als Ausbuchtung mit zwei aus dem Lichtleiter herausragenden, zueinander hin geneigten Flächen. Die oberen vier Varianten mit rückwärtigen Eintrittsprismen 8 bieten den Vorteil, dass sie weniger anfällig für Verschmutzung sind als die unterste Variante mit einem vorderseitigen Eintrittsprisma 23. Diese Strukuren gemäss der **Figur 7** können aus einzelnen grossen oder makroskopischen Prismen bestehen, und/oder aus einer Vielzahl von Mikroprismen, wie beispielsweise in der US 2001/053075 A1 beschrieben.

**Figur 8** zeigt verschiedene weitere Formen von Austrittsprismen 7, und zwar, von oben nach unten: Ein Prisma 7 mit gegenüberliegenden, zueinander hin geneigten Flächen, welche im Lichterfassungselement 4 eingeschlossen und dadurch vor Verschmutzung geschützt sind; ein Prisma mit gegenüberliegenden, zueinander hin geneigten Flächen, die nach oben offen sind;

sowie ein Prisma an der dem Sensor 5 zugewandten Seite mit zwei aus dem Lichtleiter herausragenden, zueinander hin geneigten Flächen, die also eine Ausbuchtung 24 als Austrittsprisma bilden. Auch hier können makroskopische Elemente und/oder eine Vielzahl von Mikroprismen eingesetzt sein. In der mittleren Variante ist die Lichtaustrittsfläche vor dem Sensor 5 durch eine Ausformung 6 abgesetzt, so dass das Licht durch die Ausformung durch eine Öffnung in einer Printplatte oder einem Träger 18 einer elektronischen Schaltung hindurch zum Sensor 5 geführt wird. Der Sensor 5 ist dabei vorzugsweise an der Printplatte selber befestigt. Eine solche Ausformung 6 kann natürlich auch mit den anderen Varianten der Austrittsprismen kombiniert werden.

**Figur 9** zeigt verschiedene Ecken 19 an Lichterfassungselementen 4. Das Lichterfassungselement 4 weist eine längliche und um mindestens eine Ecke 19 führende Form auf. Dabei ist der Aussenbereich der Ecke 19 zum Umlenken des Lichtes innerhalb des Lichterfassungselementes 4 gerundet oder weist eine Fläche 20 senkrecht zur Winkelhalbierenden der Ecke 19 auf. Licht das im einen Schenkel erfasst wird, wird so um die Ecke 19 in den anderen Schenkel zu beispielsweise einem Sensor 5 geführt.

**Figur 10** zeigt eine seitliche Ansicht eines Teils einer Blendschutzeinheit. Die Orientierung entspricht der Orientierung im bestimmungsgemässen Betrieb; die Blickrichtung der Sensoren 5 ist im wesentlichen horizontal, und die Blendschutzkassette 1 und das Filter 3 sind etwa senkrecht ausgerichtet. Die Lichteintrittsfläche 12 des Lichterfassungselementes 4 ist bezüglich des Sensors 5 nach unten verschoben, oder zumindest durch einen Rand einer Abdeckung entsprechend asymmetrisch abgedeckt. Dadurch wird dem Sensor 5 vor allem Licht von unten her zugeführt. Dadurch wird der Einfluss von störendem Umgebungslicht verringert.

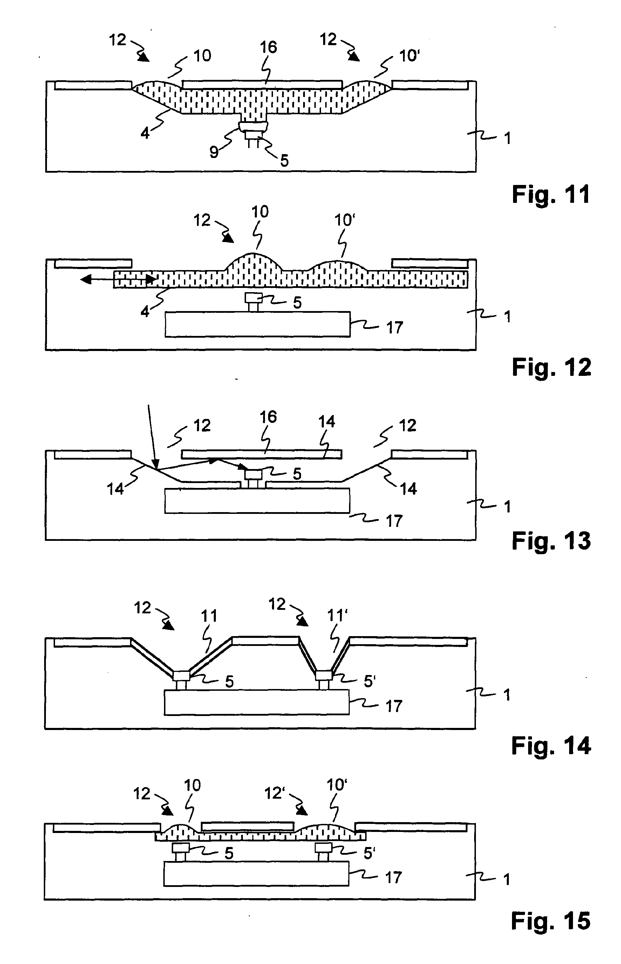

**Figur 11** zeigt eine Ausführungsform der Erfindung mit angeformten Linsen 10 und teilweise abgedecktem Lichterfassungselement 4 in einem seitlichen Querschnitt. Hier ist ein Teil des Lichterfassungselementes 4 durch eine Abdeckung 16 abgedeckt, so dass nur zwei Bereiche als Erfassungsbereiche 12 wirken. Die konvexe Hülle der Erfassungsbereich 12 (von oben gesehen) umspannt einen wesentlich grösseren Bereich als die Sensorfläche selber. Damit wird die Lichterfassung unempfindlich gegenüber lokalen Schatten oder einer lokalen Verdeckung.

Die Linsen 10 sind vorzugsweise eine rotationssymmetrische Oberfläche auf, sind also beispielsweise sphärisch oder asphärisch. In einer anderen Ausführungsform der Erfindung sind die Linsen zylindrisch, und erstreckt sich eine solche zylindrische Linsenoberfläche in der Längsrichtung des Lichterfassungselementes 4.

**Figur 12** zeigt eine Lichterfassung mit verschiebbaren Linsen 10, 10'. Ein tragendes Element oder Linsenträger 13 ist zusammen mit den Linse 10 beweglich in der Blendschutzeinheit 1 angeordnet, so dass eine erste oder eine zweite der Linsen 10, 10' vor den Sensor 5 geschoben werden kann. Die beiden Linsen weisen unterschiedliche optische Charakteristiken, insbesondere Brennweiten auf, so dass der effektive Sichtwinkel oder Öffnungswinkel des Sensors 5 durch Wahl der Linse bestimmt ist.

**Figur 13** zeigt eine Ausführungsform mit reflektierenden Oberflächen. Hier liegt kein Körper vor, durch den das Licht geleitet wird, sondern das Licht wird durch einen innenverspiegelten Hohlleiter oder einen Hohlraum mit spiegelnden Oberflächen 14 von den Erfassungsbereichen 12 zum Sensor 5 geführt.

**Figur 14** zeigt eine Lichtführung durch den Sensoren 5, 5' vorgelagerte Blenden mit Blendenrändern 11, 11', welche den Blickwinkel der Sensoren 5, 5' mehr oder weniger begrenzen. **Figur 15** zeigt eine alternative Lichtführung durch jeweils den Sensoren fest zugeordnete Linsen 10, 10'. In den Ausführungsform der Erfindung gemäss den **Figuren 14** und **15** geschieht die Auswahl unterschiedlicher Blickwinkel durch Umschalten zwischen den Sensoren 5, 5'.

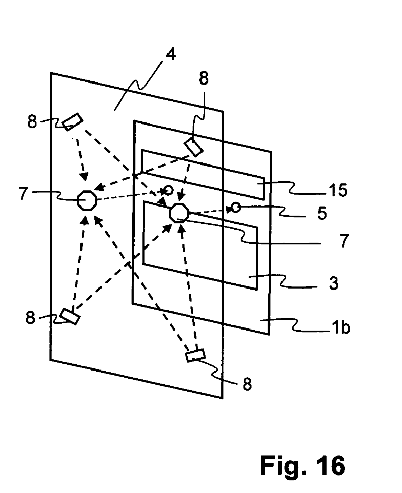

**Figur 16** zeigt eine bevorzugte Ausführungsform der Erfindung mit einem Lichterfassungselement 4, welches getrennt und ausserhalb von der Blendschutzkassette angeordnet ist. Die Blendschutzeinheit 1 besteht deshalb aus einer Blendschutzkassette als Basis-Blendschutzeinheit 1b, und dem Lichterfassungselement 4, wobei das Lichterfassungselement 4 Teil einer anderer anderen Komponente der Schutzmaske 2 ist, insbesondere Teil einer Schutzscheibe 22. Die Basis-Blendschutzeinheit 1b ist also gleich aufgebaut wie die bisher beschriebene Blendschutzeinheit 1, mit der Ausnahme, dass das Lichterfassungselement 4 nicht Teil der Basis-Blendschutzeinheit 1b selber ist. In der **Figur 16** ist der Verlauf des erfassten Lichtes zu den Sensoren 5 schematisch durch strichlierte Pfeile gezeigt. Zu Zwekken der besseren Illustration ist dabei der Abstand zwischen dem Lichterfassungselement 4 und der Basis-Blendschutzeinheit 1b übertrieben gross dargestellt. In einer bevorzugten Ausführungsform der Erfindung ist der Abstand zwischen dem Lichterfassungselement 4 im Bereich der Austrittsprismen 7 und den Sensoren 5 so klein wie möglich und vorzugsweise mindestens annähernd Null. Das Lichterfassungselement 4 weist auch in dieser Ausführungsform der Erfindung Eintrittsprismen 8 und Austrittsprismen 7 auf, deren Wirkungsweise dieselbe wie bei den bisherigen Ausführungsformen ist.

Die Austrittsprismen 7 sind korrespondierend zu den Sensoren 5 der Basis-Blendschutzeinheit 1b angeordnet, d.h. derart dass im fertig montierten Betriebszustand des Lichterfassungselementes 4 resp. der Schutzscheibe 22 und der Basis-Blendschutzeinheit 1b in der Maske die Austrittsprismen 7 den Sensoren 5 gegenüberliegen und Licht zu den Sensoren 5 leiten. Die Austrittsprismen 7 weisen jeweils angewinkelte Flächen auf, die gegen zugeordnete Eintrittsprismen 8 gerichtet sind und so Licht aus verschiedenen Richtungen zum jeweiligen Sensore 5 reflektieren. Das Lichterfassungselement 4 kann auch Ausformungen 6 wie weiter oben beschrieben aufweisen.

Die Eintrittsprismen 8 sind analog zu jenen der bisherigen Ausführungsformen geformt. Sie können aber zusätzlich auch, in der Aufsicht auf die Fläche des Lichterfassungselementes 4 gesehen, gekrümmt sein oder aus mehreren zueinander im Winkel stehenden Abschnitten bestehen, so dass jeweils eines der Eintrittsprismen 8 Licht zu mehr als einem der Austrittsprismen 7 leitet. Die Eintrittsprismen 8 können in einer linearen Anordnung wie in der **Figur 3** oder aber, wie in der Figur 16, über die Fläche des Lichterfassungselementes 4 verteilt sein, welche in diesem Fall vorzugsweise identisch mit der Schutzscheibe 22 oder einer Teilfläche der Schutzscheibe 22 ist. Alternativ kann aber das Lichterfassungselement 4 auch ein- oder mehrstückig separat von einer Schutzscheibe 22 geformt und auf eine Schutzscheibe geklebt oder zwischen der Schutzscheibe 22 und der Basis-Blendschutzeinheit 1b angeordnet sein.

**BEZUGSZEICHENLISTE**

| | |
|---|---|
| 1 | Blendschutzeinheit |
| 1b | Basis-Blendschutzeinheit |
| 2 | Schutzmaske |
| 3 | Filter |
| 4 | Lichterfassungselement |
| 5, 5', 5' | Sensor |
| 6 | Ausformung |
| 7 | Austrittsprisma |
| 8 | Eintrittsprisma |
| 9 | Klebstoff |
| 10 | Linse |
| 11 | Blendenrand |
| 12 | Erfassungsbereich |
| 13 | Linsenträger |
| 14 | spiegelnde Oberflächen |
| 15 | Solarzelle |
| 16 | Abdeckung |
| 17 | Steuerelektronik |
| 18 | Schaltungsträger |
| 19 | Ecke |
| 20 | Eckfläche |
| 21 | Ablenkfläche |
| 22 | Schutzscheibe |
| 23 | Ausbuchtung zum Einkoppeln |
| 24 | Ausbuchtung zum Auskoppeln |

**Patentansprüche**

1. Blendschutzeinheit (1) für eine tragbare Blendschutzvorrichtung, wobei die Blendschutzeinheit (1) aufweist ein optisches Blendschutzfilter (3), mindestens einen Sensor (5) zur Erfassung von einfallendem Licht und zur Ansteuerung der Durchlässigkeit des Filters (3) nach Massgabe der erfassten Lichtmenge, **dadurch gekennzeichnet, dass** die Blendschutzeinheit (1) ferner aufweist ein Lichterfassungselement (4), welches einfallendes Licht auf einem oder mehreren Erfassungsbereichen (12) erfasst und durch Mittel zum Umlenken von Licht dem mindestens einen Sensor (5) zuführt, wobei die Fläche des oder der Erfassungsbereiche (12) grösser ist als die Fläche des mindestens einen Sensors (5) und wobei Mittel (8) zum Einkoppeln von Licht in das Lichterfassungselement (4) voneinander beabstandet sind.

2. Blendschutzeinheit (1) gemäss einem der vorherigen Ansprüche, in welcher einem Lichterfassungselement (4) zwei oder mehr Sensoren (5, 5') zugeordnet sind.

3. Blendschutzeinheit (1) gemäss Anspruch 2, wobei das Lichterfassungselement (4) einstückig geformt ist, und am Lichterfassungselement (4) für zwei oder mehr Sensoren (5, 5') jeweils ein eigenes Mittel zum Auskoppeln des erfassten Lichtes zum jeweiligen Sensor (5, 5') ausgebildet ist.

4. Blendschutzeinheit (5) gemäss einem der Ansprüche 1 bis 3, in welcher das Lichterfassungselement (4) als lichtleitende Strukturen Einbuchtungen oder Hohlräume (7, 8) aufweist, und die Umlenkung des Lichtes unter anderem durch interne Reflexion innerhalb des Lichterfassungselementes (4) geschieht.

5. Blendschutzeinheit (5) gemäss Anspruch 4, wobei die Strukturen zum Einkoppeln (6) von Licht in das Lichterfassungselement (4) einen Abstand von mindestens dem doppelten der lokalen Dicke des Lichterfassungselementes (4) zueinander aufweisen.

6. Blendschutzeinheit (5) gemäss einem der vorherigen Ansprüche, in welcher durch mindestens eine Einbuchtung oder einen Hohlraum (7) Licht aus zwei entgegengesetzten Richtungen aus dem Lichterfassungselement (4) zum selben Sensor (5) ausgekoppelt wird, und das Lichterfassungselement (4) im Bereich dieser Einbuchtung respektive dieses Hohlraums (7) einstückig geformt ist.

7. Blendschutzeinheit (1) gemäss einem der vorherigen Ansprüche, in welcher die Mittel zum Umlenken Austrittsprismen (7) und/oder Eintrittsprismen (8) sind, welche sich von einem äusseren Teil des Lichterfassungselementes (4) her in das Lichterfassungselement (4) hinein erstrecken.

8. Blendschutzeinheit (1) gemäss Anspruch 7, in welcher mindestens eines der Eintrittsprismen (8) mindestens eine Ablenkfläche (21) aufweist, die gegenüber einer Referenzeintrittsrichtung um einen Neigungswinkel von 0 bis 90° und insbesondere von 10° bis 70° und vorzugsweise von 35° bis 50° geneigt ist, wobei die Referenzeintrittsrichtung parallel zu einer Flächennormalen einer lichterfassenden Oberfläche des Lichterfassungselementes (4) ist.

9. Blendschutzeinheit (1) gemäss Anspruch 8, in welcher mindestens eines der Eintrittsprismen (8) eine Ablenkfläche (21) mit einem ersten Neigungswinkel und mindestens ein weiteres der Eintrittsprismen (8) eine Ablenkfläche (21) mit einem zweiten, unterschiedlichen Neigungswinkel aufweist.

10. Blendschutzeinheit (1) gemäss einem der Ansprüche 7 bis 9, in welcher mindestens ein Austrittsprisma (7) am Lichterfassungselement (4) einem der mindestens einen Sensoren (5) gegenüber angeordnet ist, und eines oder mehrere der Eintrittsprismen (8) entlang des Lichterfassungselementes (4) auf derselben Seite des Lichterfassungselementes (4) wie der mindestens eine Sensor (5) angeordnet sind, und von dem mindestens einen Sensor (5) beabstandet angeordnet sind.

11. Blendschutzeinheit (1) gemäss einem der Ansprüche 7 bis 10, wobei mindestens eines der Austrittsprismen (7) abgedeckt respektive im Lichterfassungselement (4) eingeschlossen ist.

12. Blendschutzeinheit (5) gemäss einem der vorherigen Ansprüche, in welcher das Lichterfassungselement (4) als lichtleitende Strukturen Ausbuchtungen (23, 24) aufweist.

13. Blendschutzeinheit (1) gemäss einem der vorherigen Ansprüche, in welcher das Lichterfassungselement (4) mindestens eine Ausformung (6) aufweist, welche das Licht durch einen Träger (18) einer elektronischen Schaltung (17) hindurch zum Sensor (5) führt.

14. Blendschutzeinheit (1) gemäss einem der vorherigen Ansprüche, in welcher das Lichterfassungselement (4) ein linear ausgedehntes oder stabartiges Prisma aus einem lichtdurchlässigen Material ist.

15. Blendschutzeinheit (1) gemäss Anspruch 14, in welcher das Lichterfassungselement (4) sich linear entlang einer Seite des Filters (3) erstreckt, und insbesondere entlang mindestens zwei Dritteln dieser Seite.

16. Blendschutzeinheit (1) gemäss einem der vorherigen Ansprüche, in welcher das Lichterfassungselement (4) eine längliche und um mindestens eine Ecke (19) führende Form aufweist, wobei der Aussenbereich der Ecke (19) zum Umlenken des Lichtes innerhalb des Lichterfassungselementes (4) gerundet ist oder eine Fläche (20) senkrecht zur Winkelhalbierenden der Ecke (19) aufweist.

17. Blendschutzeinheit (1) gemäss einem der vorherigen Ansprüche, in welcher das Lichterfassungselement (4) entlang einer im vorgesehenen Betrieb annähernd senkrechten Achse bezüglich der Sensoren (5, 5') asymmetrisch und insbesondere nach unten verschoben angeordnet ist.

18. Blendschutzeinheit (1) gemäss einem der vorherigen Ansprüche, in welcher der mindestens eine Sensor (5) durch einen Klebstoff (9) am Lichterfassungselement (4) angeklebt ist, wobei der Klebstoff (9) mindestens annähernd denselben Brechungsindex wie das Material des Lichterfassungselementes (4) aufweist.

19. Blendschutzeinheit (1) gemäss einem der vorherigen Ansprüche, in welcher das Lichterfassungselement (4) im Erfassungsbereich (12) als Linse (10) ausgebildet ist.

20. Blendschutzeinheit (1) gemäss einem der vorherigen Ansprüche, aufweisend eine Blendschutzkassette als Basis-Blendschutzeinheit (1b) mit dem Filter (3) und dem mindestens einen Sensor (5), wobei das Lichterfassungselement (4) ausserhalb der Basis-Blendschutzeinheit (1b) angeordnet ist und bei einer betriebsbereiten Anordnung in der Blendschutzvorrichtung (2) eine definierte Position gegenüber dem mindestens einen Sensor (5) aufweist und das einfallende Licht dem mindestens einen Sensor (5) zuführt.

21. Blendschutzeinheit (1) gemäss Anspruch 20, in welcher das Lichterfassungselement (4) in eine transparente Schutzscheibe (22) integriert ist, und insbesondere einstückig mit der Schutzscheibe geformt ist.

22. Ersatzteil, insbesondere eine transparente Schutzscheibe (22), zur Verwendung in einer tragbaren Blendschutzvorrichtung (2) mit einer Blendschutzeinheit (1) gemäss Anspruch 21 oder 22, wobei das Ersatzteil aufweist ein Lichterfassungselement (4), welches bei einer betriebsbereiten Anordnung in der Blendschutzvorrichtung (2) eine definierte Position gegenüber dem mindestens einen Sensor (5) aufweist und das einfallende Licht dem mindestens einen Sensor (5) zuführt.

**Fig. 1**

**Fig. 2**

**Fig. 3**

**Fig. 4**

**Fig. 5**

**Fig. 7**

**Fig. 6**

**Fig. 8**

**Fig. 9**

**Fig. 10**

**Fig. 11**

**Fig. 12**

**Fig. 13**

**Fig. 14**

**Fig. 15**

**Fig. 16**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20050007667 A1
- US 20050097648 A1
- US 2001053075 A1